# EUROPEAN PATENT SPECIFICATION

(11) **EP 0 984 976 B1**
(45) Date of publication and mention of the grant of the patent: **02.10.2002**
(21) Application number: 98934903.0
(22) Date of filing: 21.05.1998
(51) Int. Cl.: C07H 19/06

(54) **PROCESS FOR THE PREPARATION OF A DEOXYURIDINE DERIVATIVE**
VERFAHREN ZUR HERSTELLUNG VON DEOXYURIDINDERIVATE
PROCEDE DE PREPARATION D'UN DERIVE DE DEOXYURIDINE

(30) Priority: 23.05.1997 IT MI971211
(43) Date of publication of application: 15.03.2000
(73) Proprietor: PRO.BIO.SINT. S.r.l., 21100 Varese (IT)
(72) Inventor: COTTICELLI, Giovanni, I-20063 Cernusco Sul Naviglio (IT); DE MEGLIO, Giuseppe, I-20129 Milano (IT); MONCIARDINI, Simone, I-21100 Varese (IT); ORDANINI, Giancarlo, I-21039 Bedero Valcuvia (IT)
(74) Representative: Dragotti, Gianfranco
(86) International application number: EP9803171
(87) International publication number: WO98052960

(56) References cited:
- US-A- 4 071 680
- US-A- 4 340 729
- S. AJMERA & P. V. DANENBERG: "Synthesis and biological activity of 5'-substituted 5-fluoropyrimidine nucleosides" JOURNAL OF MEDICINAL CHEMISTRY, vol. 25, no. 8, August 1982, pages 999-1002, XP002037737 WASHINGTON US

## Description

The present invention relates to a process for the preparation of a deoxyuridine derivative. More particularly, the invention concerns a process for the preparation of 5'-deoxy-5-fluorouridine as well as novel intermediates useful in this process.

The compound 5'-deoxy-5-fluorouridine is a well known cytostatic agent which will be hereinafter designated by its International Non-proprietary Name *doxifluridine*.

Doxifluridine, having the formula I, 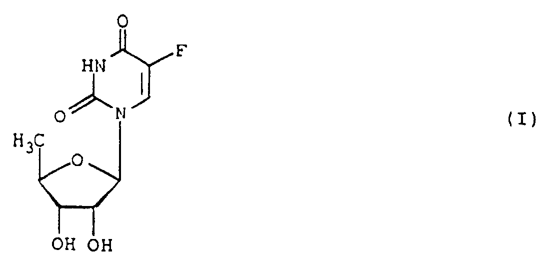 is described in US Patent 4,071,680, wherein a multi-step synthesis, starting from 5-fluorouridine and involving the removal of the 5'-hydroxy group through the corresponding 5'-iododerivative, is also disclosed. According to this document, the replacement of the 5'-hydroxy group by a iodine atom is accomplished by using triphenylphosphite methoiodide as a chemical iodinating agent which requires a particular caution because of the toxicity of the reagent owing to the presence of reaction by-products.

EP 21,231 discloses the preparation of doxifluridine by removal of the acyl groups from the corresponding 2',3'-diesters with carboxylic acids, particularly from the corresponding 2',3'-diacetate. However, the synthesis of the starting material involves the replacement of an hydroxy group by a bromine atom and the conversion of the bromo derivative into the corresponding deoxy compound by catalytic hydrogenation. In this case too, the bromination is carried out by using a phosphor compound, namely with bromine in the presence of a great amount of triphenylphosphine.

According to a paper by S. Aymera and P.V. Danenberg (J. Med. Chem. 1982, 25, 999), 5'-deoxy-5'-iodo-2',3'-O-isopropylidene-5-fluoro uridine is prepared according to the method of Cook et al. (J. Med. Chem. 1979, 22, 1330), which corresponds to US 4,071,680. The same paper discloses the conversion of 5'-deoxy-5'-O-mesyloxy-2',3'-O-isopropylidene-5-fluorouridine into 5'-bromo-5'-deoxy-2',3'-O-isopropy lidene-5-fluorouridine by lithium bromide, but it does not describe any further conversion.

On the other hand, in the above cited patent EP 21,231, the replacement of a bromine atom by a hydrogen atom is conducted by catalytic hydrogenation under strong conditions, i.e. in the presence of potassium hydroxide, on a substrate which does not contain the uracil moiety, this one being introduced after the preparation of the deoxy-sugar in the presence of a strong inorganic base.

It has now been found that it is possible to replace the hydroxyl group of 2',3'-isopropylidene-5-fluorouridine by a iodine atom by simply using sodium iodide, in the absence of any phosphor compound, if said hydroxy group is previously activated as a sulfonic ester.

It has also been found that the iodine atom of 5'-deoxy-5'-iodo-2',3'-O-isopropylidene-5-fluorouridine can be replaced by a hydrogen atom by catalytic hydrogenation without using strong bases, thus obtaining the corresponding 5'-deoxy compound in very good yields. The same replacement can take place with other hydrogen donors such as cyclohexene, cyclohexadiene or an hydride, for example tributyltin hydride.

Thus, it is an object of the present invention to provide a process for the preparation of 5'-deoxy-5-fluorouridine, which comprises:
(a) reacting 2',3'-O-isopropylidene-5-fluorouridine of formula II 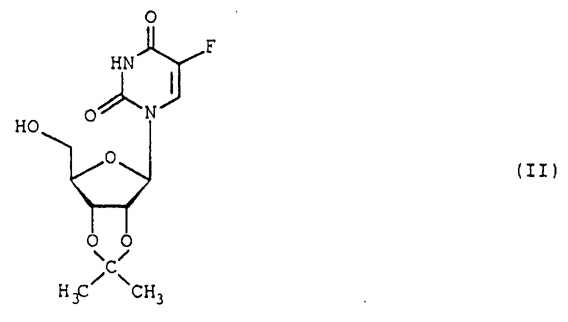 with a functional derivative of a sulfonic acid of formula III

   R―SO₃H (III)

   wherein R is a (C₁-C₄)alkyl, a trifluoromethyl, an unsubstituted, mono- di- or trisubstituted phenyl group;
(b) reacting the 5'-sulfonyloxy derivative thus obtained of formula IV 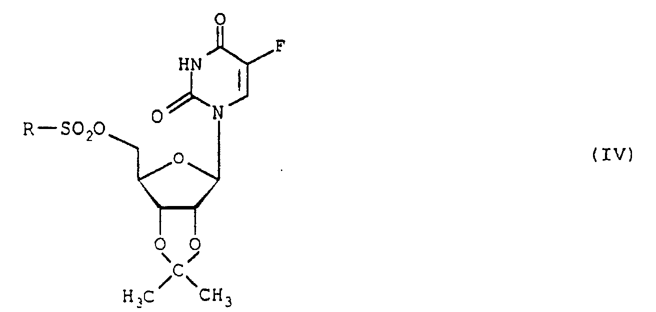 wherein R is as defined above, with an alkaline or earth-alkaline iodide;
(c) hydrolysing the 5'-deoxy-5'-iodo-2',3'-O-isopropylidene-5-fluorouridine of formula V 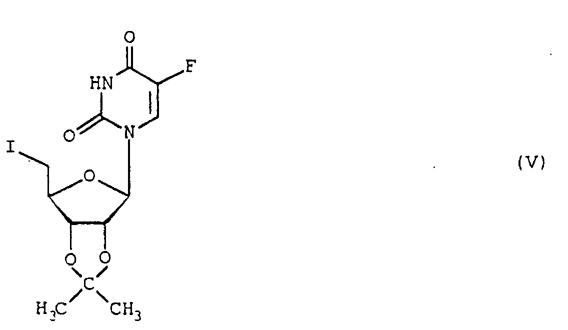 thus obtained in acidic medium; and
(d) submitting the 5'-deoxy-5'-iodo-5-fluorouridine of formula VI 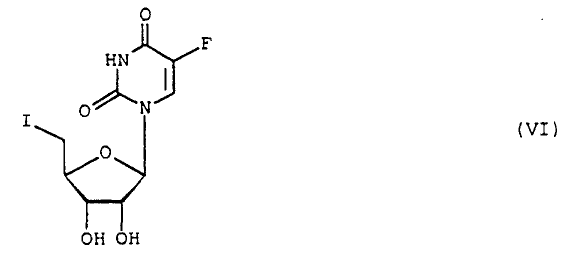 to a reduction with hydrogen or a hydrogen donor.

In formula III R is preferably methyl, ethyl, n-butyl, trifluoromethyl, phenyl, monosubstituted phenyl, i.e. with a methyl, methoxy, nitro group or halogen, disubstituted, i.e. with two methyl groups, or trisubstituted, i.e. with three methyl groups, particularly 2,4,6-trisubstituted.

As a functional derivative of the sulfonic acid, the chloride, the anhydride or a mixed anhydride is suitably used. Methanesulfonyl chloride, benzenesulfonyl chloride, p-toluenesulfonyl chloride, trifluoromethanesulfonyl chloride and 2,4,6-trimethylphenylsulfonyl chloride are particularly preferred as esterifying agents.

Thus, in step (a), the functional derivative of the sulfonic acid of formula III, preferably selected from the group consisting of those defined hereinabove, is made to react with 2',3'-O-isopropylidene-5-fluorouridine. Advantageously, the reaction is carried out in an organic solvent, i.e. halogenated such as dichloromethane, 1,2-dichloroethane or 1,1,1-trichloroethane, or a polar aprotic solvent, such as N,N-dimethylformamide, N,N-dimethylacetamide, dimethyl sulfoxide or acetonitrile, or ethyl acetate or an aromatic hydrocarbon such as toluene or xylene, in the presence of a base such as trimethylamine, triethylamine, diisopropylamine, N-ethyl-diisopropyl amine, pyridine or dimethylaminopyridine.

Generally, after 5ö6 hours at a temperature of 0ö+40°C, the reaction is complete and the compound of formula IV thus obtained is separated from the reaction by-products by simple filtration of said by-product, after treatment with water.

The 5'-sulfonyloxy derivative of formula IV may be recovered by extraction with an organic solvent, subsequent concentration and may be isolated and characterized according to conventional methods.

Alternatively, the concentrated solution of the compound of formula IV may be straightforwardly used for step (b).

Step (b) is carried out by simply treatment of the compound of formula IV, in pure form or as the said concentrated solution obtained at the end of step (a) , with an alkaline or earth-alkaline iodide in an organic solvent such as a ketone, preferably acetone, methylethyl ketone or methylisobutylketone, an ether, preferably dioxane or tetrahydrofurane, or a polar aprotic solvent, preferably acetonitrile, N,N-dimethylformamide, N,N-dimethylacetamide or dimethylsulfoxide.

After 4ö6 hours at a temperature of 40ö80°C, the reaction is complete and, as in the step (a), the by-products of the reaction are removed by filtration and by washing with water. The expected end product, namely 5'-deoxy-5'-iodo-2',3'-O-isopropylidene-5-fluoro uridine of formula V may be recovered with an organic solvent, preferably ethyl acetate, subsequent concentration, and may be isolated according to conventional methods. As in step (a), the concentrated solution containing the compound of formula V may be directly used for step (c).

Step (c) consists of a hydrolysis of 5'-deoxy-5'-iodo-2',3'-O-isopropylidene-5-fluorouridine in acidic medium according to the methods commonly used in the sugar chemistry and, more particularly, in that of the nucleosides. Preferably such an hydrolysis is carried out in aqueous formic acid or, more advantageously, in aqueous acetic acid, or in N,N-dimethylformamide or N,N-dimethylacetamide in the presence of aqueous HCl, at a temperature of 80ö100°C.

The 5'-deoxy-5'-iodo-5-fluorouridine of formula VI thus obtained is isolated by evaporation of the solvent and the compound is recovered by extraction from a suitable solvent, preferably ethyl acetate.

As set forth hereinabove, the compounds obtained at the end of steps (a) and (b) may be isolated and characterized, or, preferably, they may be used, without isolation, in a raw state or dissolved or suspended in a solvent. The yields of the three steps are very high, namely always higher than 90% of the theoretical and compound of formula VI is recovered in yield as high as 83ö85%, calculated on the starting 2',3'-O-isopropylidene-5-fluorouridine of formula II.

In step (d) the compound of formula VI is subjected to a reduction, which may be carried out by catalytic hydrogenation or by using cyclohexene or cyclohexadiene as hydrogen donors, for example in the presence of Pd/C, preferably at 5%, as a catalyst, or also by a hydride.

The reduction is carried out in an organic solvent, for example in an alcohol, such as methanol, ethanol, propanol, isopropanol or n-butanol or in a mixture thereof. Preferably, an organic base, such as trimethylamine, triethylamine, diisopropylamine, N-ethyldiisopropyl amine, pyridine, dimethylaminopyridine, morpholine, N-methyl morpholine, 2-picoline and quinoline or an inorganic base such as an alkaline bicarbonate, for example sodium bicarbonate or potassium bicarbonate, is present in the reaction mixture.

When the reduction is conducted with hydrogen, the hydrogenation occurs at room pressure or at 1ö2 bar and at room temperature, in the presence of 5% Pd/C.

When the reduction is carried out in the presence of a hydrogen donor, such as cyclohexene, ciclohexadiene or a hydride, for example tributyltin hydride, the reaction takes place in an alcoholic solvent such as methanol, ethanol, isopropanol, n-butanol, isobutanol and the like, or in mixtures of said solvents with an aprotic solvent, such as toluene.

More particularly, when the reduction is conducted with ciclohexene or cyclohexadiene as a hydrogen donor, at a temperature of 60ö90°C, is completed after 3ö6 hours.

The 5'-deoxy-5-fluorouridine thus obtained is isolated according to conventional methods, more particularly by filtering the catalyst and evaporating the solvent. The end product is crystallized with a mixture of ethanol/isopropanol = 3/2 (v/v).

When the reduction is carried out by using tributyltin hydride, the reaction takes place by dissolving 5'-deoxy-5'-iodo-5-fluorouridine in an alcohol, for example methanol, the reducing agent being added as a solution in an organic solvent, for example in toluene, and the mixture is heated at reflux in the presence of α,α'-azoisobutyro nitrile in catalytic amount. After 1ö3 hours the reduction is complete and, after evaporation of the solvent, the 5'-deoxy-5-fluorouridine thus obtained is recovered according to conventional methods.

According to a preferred embodiment of the present invention, steps (a), (b) and (c) are carried out without isolating the product obtained at the end of each of steps (a) and (b). Thus, the process of the present invention allows the preparation of doxifluridine in a very easy way and in very high yields. Moreover, this process may be carried out according the *one-pot* technique as regards steps (a), (b) and (c). Said process is depicted in Scheme I, wherein R is a (C₁-C₄)alkyl, trifluoromethyl, unsubstitute or mono-, di- or trisubstituted phenyl.

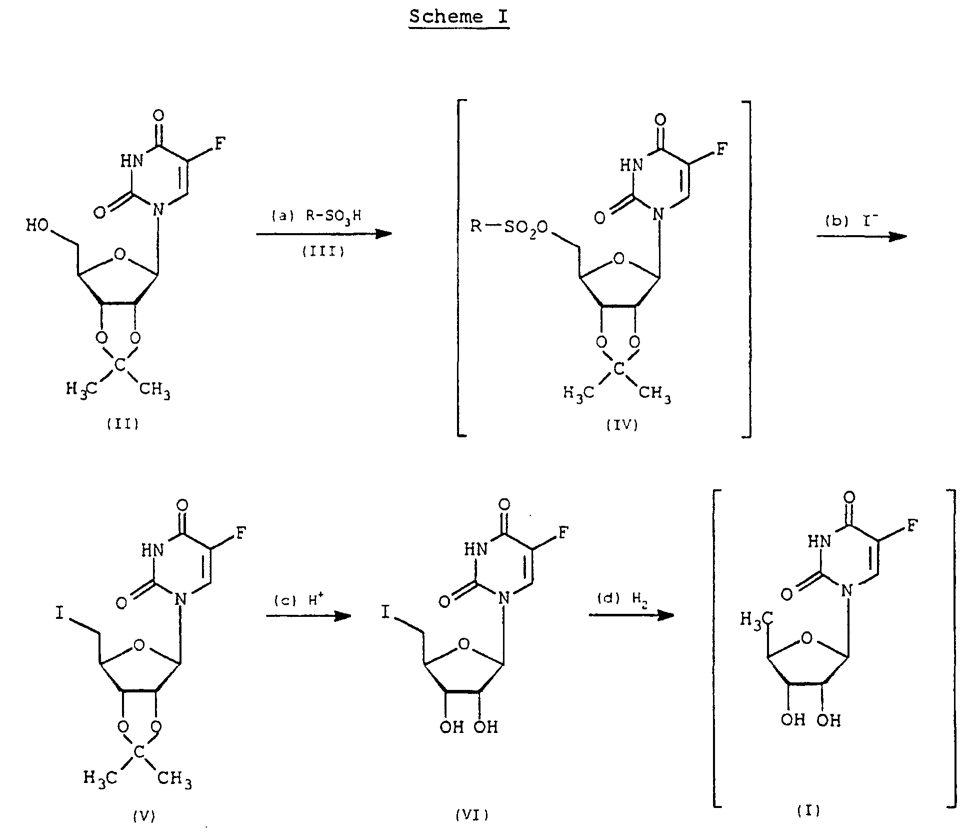

The following examples illustrate the invention without, however, limiting it.

### Example 1

To a solution of 80 g (0.26 m) of 2',3'-O-isopropylidene-5-fluoro uridine in 700 ml of pyridine, cooled to about +5°C, 90.6 g (0.48 m) of p-toluenesulfonylchloride are added. The mixture thus obtained is stirred for 5 hours, then 10 1 of water are slowly added and the aqueous suspension is stirred for 3 hours. The mixture is let to stand at room temperature for 15 hours, then the precipitate is filtered, washed with water and dried. Thus, 99.2 g (83%) of 2',3'-O-isopropyli dene-5'-O-(p-toluenesulfonyl)-5-fluorouridine in crystalline form is obtained. M.p. = 154ö156°C; purity (HPLC) = 99.89% and [α]_{D} = +26,71° (c = 1, CH₃OH).

The product thus obtained may be purified by cystallization with ethanol affording a white crystalline powder. M.p. = 156ö158°C; purity = 99.9ö100% (HPLC).

### Example 2

### A. 5'-deoxy-5'-iodo-5-fluorouridine

To a solution of 39.2 g (0.13 m) of 2',3'-O-isopropylidene-5-fluorouridine in 55 ml of methylene chloride and 159 ml (1.98 m) of pyridine, cooled to +5°C, 60 g (0.315 m) of p-toluenesulfonyl chloride are added. The mixture thus obtained is stirred for 5 hours observing the formation of a plentiful precipitate. Then 160 ml of methylene chloride and 160 ml of water are added thereinto, whereby the temperature rises to 40°C. The phases are separated and the organic one is extracted with about 900 ml of N HCl; then the organic phase is washed with 2 x 100 ml of water. The organic phase, containing 0.117 m of 2',3'-O-isopropylidene-5'-O-(p-toluenesulfonyl)-5-fluorouridine (yield - 90% of the theoretical), is concentrated under vacuum until an oily residue is obtained, which is taken up with 2 x 100 ml of acetone. The oily residue is dissolved with 600 ml of acetone and 105 g (0.70 m) of sodium iodide are added to the solution. The mixture is heated for 5 hours at reflux, then cooled, concentrated under vacuum and the residue thus obtained is taken up with 2 x 80 ml of ethyl acetate. The residual oil is treated with 800 ml of ethyl acetate and 100 ml of water. The phases are separated and the aqueous one is treated with 2 x 80 ml of ethyl acetate. The collected organic phases are washed with 2 x 100 ml of a 5% aqueous solution of sodium metabisulphite, then with 100 ml of water. The organic phase, containing 0.113 m of 5'-deoxy-5'-iodo-2',3'-O-isopropylidene-5-fluoro uridine (yield - 97% of the theoretical), is concentrated under vacuum to a small volume; to the residue 560 ml of 80% aqueous acetic acid are added and the mixture is heated to 95°C. After 4-hour heating, a check by HPLC is performed (0.34% of residual starting product). After cooling, the mixture is concentrated under vacuum until an oily residue is obtained, which is taken up with 150 ml of ethyl acetate. The mixture is let to stand at 20ö25°C for 15 ore. The product is filtered, washed with ethyl acetate and dried under vacuum at 45°C. Thus, 44.5 g of 5'-deoxy-5'-iodo-5-fluorouridine are obtained. M.p. = 174ö175°C, purity (HPLC) = 99.74% and [α]_{D} = +8.96° (c = 1, CH₃OH). The mother liquors are concentrate under vacuum and the residue is taken up with 50 ml of ethyl acetate; after 15 hours at +5°C, a further amount of 3.3 g of product are obtained. Total yield: 83% of the theoretical.

### B. 5'-deoxy-5-fluorouridine

To a suspension of 20 g (0.053 m) of 5'-deoxy-5'-iodo-5-fluoro uridine in a mixture of 60 ml of ethanol and 40 ml of isopropanol, 15.2 ml (0.108 m) of diisopropylamine are added, then 3 g of 5% Pd/C are added thereinto and the mixture is hydrogenated at about 1-bar pressure for 14 hours. After removal of the catalyst by filtration, the solution is concentrated under vacuum until a solid residue is obtained, which is crystallized with a mixture ethanol/isopropanol = 60/40 v/v; after 8 hours the product is filtered, washed with said mixture and dried at 45°C for 15 hours. Thus, 11.6 g (yield - 89% of the theoretical) of 5'-deoxy-5-fluorouridine. M.p. = 187ö188°C, puritya (HPLC) = 99.75% and [α]_{D} = +18.2° (c = 0.42, H₂O).

### Example 3

To a solution of 40 g (0.087 m) of 2',3'-O-isopropylidene-5'-O-(p-toluenesulfonyl)-5-fluorouridine, obtained as described in Example 1, in 1700 ml of acetone, 105 g of sodium iodide are added and the clear solution thus obtained is heated at reflux for 4ö5 hours. Then, the mixture is cooled, filtered and washed with acetone. The collected acetonic solutions are concentrated under vacuum and the residue is taken up with 3500 ml of ethyl acetate. After washing with 500 ml of 3% aqueous solution of sodium metabisulphite and then with water, the solution in ethyl acetate is concentrated to a small volume. After 15 hours the precipitate is filtered and dried to give 33.2 g of 5'-deoxy-5'-iodo-2',3'-O-isopropylidene-5-fluorouridine as a white crystalline powder. M.p. = 199ö202°C, purity (HPLC) = 99,9% and [α]_{D} = -16.8° (c = 0.50, CH₃OH).

### Example 4

### A. 5'-deoxy-5'-iodo-5-fluorouridine

A suspension of 51 g (0.105 m) of 5'-deoxy-5'-iodo-2',3'-O-isopropylidene-5-fluorouridine in 500 ml of acetic acid and 120 ml of water is heated at 95°C for 90 minutes. The solution thus obtained is concentrated under vacuum and the residue is taken up with 2 1 of ethyl acetate, then is concentrated to a small volume and, after 15 hours at room temperature, the precipitate is filtered, washed with ethyl acetate and dried to give 35.6 g of 5'-deoxy-5'-iodo-5-fluoro uridine. M.p. = 174.5ö175.5°C, purity (HPLC) = 99.74% [α]_{D} = +8.96° (c = 1.00, CH₃OH).

### B. 5'-deoxy-5-fluorouridine

To a solution of 35.1 g (0.078 m) of 5'-deoxy-5'-iodo-5-fluorouridine and 2.93 g of α,α'-azoisobutyronitrile in 1100 ml of methanol, a solution of 37.4 g of tributyltin hydride in 310 ml of toluene is added. The mixture is heated at reflux for 2 hours and the clear solution thus obtained is concentrated under vacuum until a semisolid residue, which is dispersed in 900 ml of petroleum ether. The solid is filtered and treated with 1200 ml of water; the tin salts are separated by filtration and the filtrate is concentrated under vacuum; the residue is crystallized with 500 ml of hot ethanol to obtain 12.3 g di 5'-deoxy-5-fluorouridine. M.p. = 188ö189°C, purity (HPLC) = 99.91% and [α]_{D} = +18.4° (c = 0.42, H₂O).

### Example 5

To a suspension of 10 g (0.026 m) of 5'-deoxy-5'-iodo-5-fluoro uridine, obtained as described in Example 2, step A, in 100 ml of n-butanol, 6 ml of cyclohexene (0.06 m) and 14 ml (0.1 m) of triethylamine are added. The mixture is heated to 60°C in order to achieve a complete solubilization, then 1.5 g of 5% Pd/C are added thereinto. The suspension is heated at 80°C for 5 hours, filtered at the same temperature by washing with hot n-butanol and concentrated under vacuum until a residue is obtained, which crystallizes with a mixture of 60 ml of ethanol and 40 ml of isopropanol. Thus, 4.5 g of 5'-deoxy-5-fluorouridine are obtained. M.p. = 188ö189°C, purity (HPLC) = 99.87% and [α]_{D} = 18.35° (c = 0.42, H₂O).

## Claims

1. A process for the preparation of 5'-deoxy-5-fluorouridine of formula I 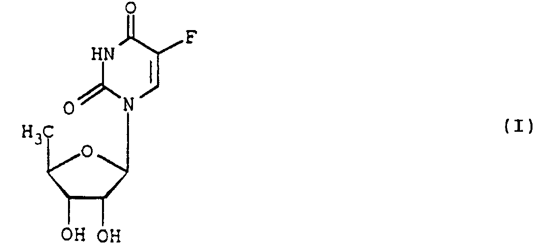 which comprises:
(a) reacting 2',3'-O-isopropylidene-5-fluorouridine of formula II 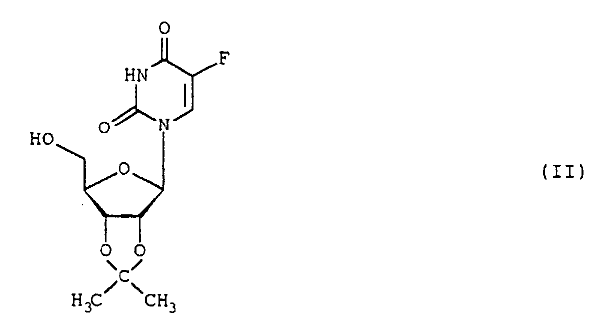 with a functional derivative of a sulfonic acid of formula III
R―SO₃H (III)
wherein R is a (C₁-C₄)alkyl, a trifluoromethyl, an unsubstituted, mono- di- or trisubstituted phenyl group;
(b) reacting the 5'-sulfonyloxy derivative thus obtained of formula IV 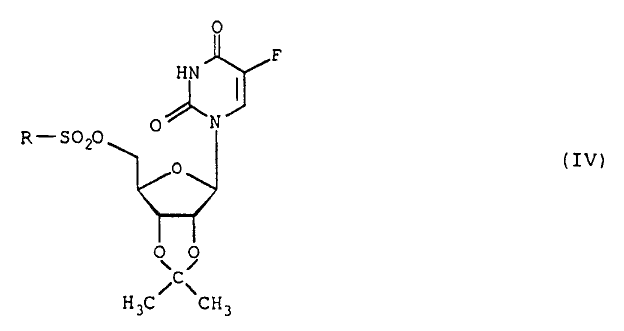 wherein R is as defined above, with an alkaline or earth-alkaline iodide;
(c) hydrolysing the 5'-deoxy-5'-iodo-2',3'-O-isopropylidene-5-fluorouridine of formula V 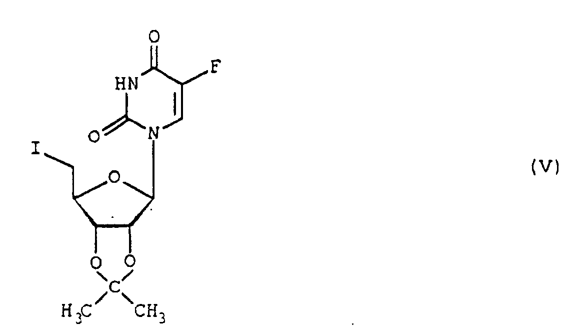 thus obtained in acidic medium; and
(d) submitting the 5'-deoxy-5'-iodo-5-fluorouridine of formula VI 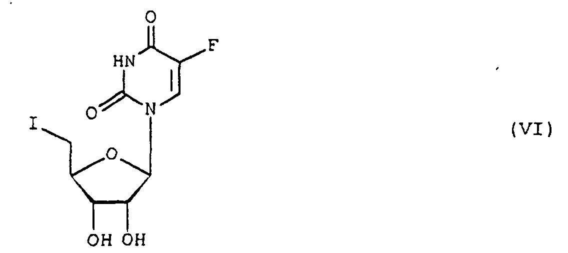 to a reduction with hydrogen or a hydrogen donor.

2. A process according to claim 1, wherein, in the said derivative of sulfonic acid of formula III, R is methyl, ethyl, n-butyl, trifluoromethyl, phenyl, monosubstituted phenyl, i.e. with a methyl, methoxy, nitro group or halogen, disubstituted, i.e. with two methyl groups, or trisubstituted, i.e. with three methyl groups, particularly 2,4,6-trisubstituted.

3. A process according to claim 1, wherein, in step (a), the chloride, the anhydride or a mixed anhydride is used as a functional derivative of the sulfonic acid of formula III.

4. A process according to claim 3, wherein the functional derivative of the acid of formula III is methanesulfonyl chloride, trifluoromethanesulfonyl chloride, benzenesulfonyl chloride, p-toluene sulfonyl chloride or 2,4,6-trimethylphenylsulfonyl chloride.

5. A process according to claim 1, wherein steps (a), (b) and (c) are carried out without isolating the products obtained at the end of each of the steps (a) and (b).

6. A process according to claim 1, wherein the reduction of step (d) is carried out by catalytic hydrogenation.

7. A process according to claim 6, wherein, palladium on charcoal is used as catalyst.

8. A process according to claim 6, wherein said catalytic hydrogenation is carried out in the presence of a base.

9. A process according to claim 8, wherein said base is a secondary or tertiary amine.

10. A process according to claim 9, wherein said secondary or tertiary amine is trimethylamine, triethylamine, diisopropylamine, N-ethyldiisopropylamine, pyridine, dimethylaminopyridine, morpholine, N-methylmorpholine, 2-picoline or quinoline.

11. A process according to claim 8, wherein said base is an inorganic base, preferably sodium or potassium bicarbonate.

12. A process according to claim 6, wherein, as reducing agent, tributyltin hydride is used.

13. A process according to claim 12, wherein the reduction is carried out in the presence of α,α'-azoisobutyronitrile, in catalytic amount.

14. A process according to claim 6, wherein the reduction is carried out using cyclohexene or cyclohexadiene as hydrogen donor.

15. A process according to claim 14, wherein said reduction step is carried out at a temperature of between 60°C and 90°C.

## Patentansprüche

1. Verfahren zur Herstellung von 5'-Desoxy-5-fluoruridin der Formel I 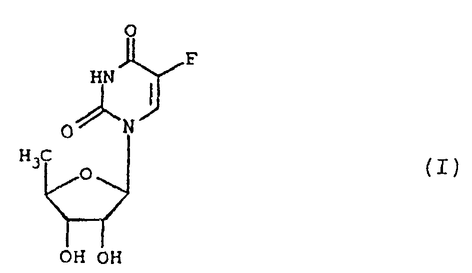 welches umfasst:
(a) Reaktion von 2',3'-O-Isopropyliden-5-fluoruridin der Formel II 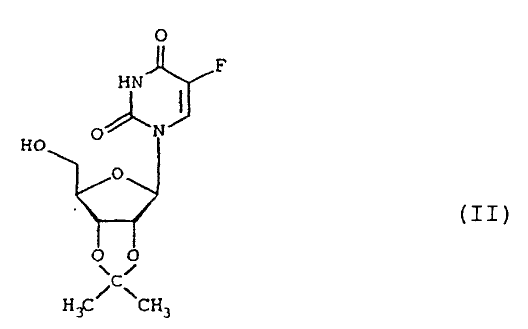 mit einem funktionellen Derivat einer Sulfonsäure der Formel III
R-SO₃H (III)
wobei R ein (C₁-C₄)-Alkyl, ein Trifluormethyl, eine nicht-substituierte, eine einfach-, zweifach- oder dreifach-substituierte Phenylgruppe ist;
(b) Reaktion des derartig erhaltenen 5'-Sulfonyloxyderivats der Formel IV 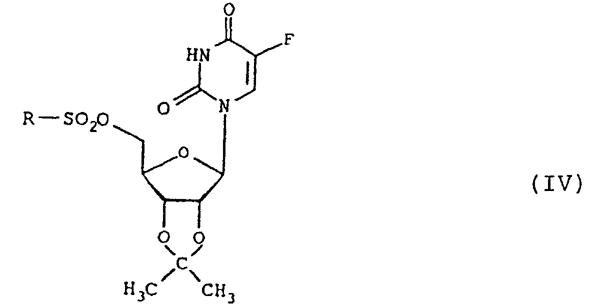 wobei R wie vorher definiert ist, mit einem Alkali- oder Erdalkaliiodid;
(c) Hydrolysieren des 5'-Desoxy-5'-iod-2',3'-Oisopropyliden-5-fluoridin der Formel V 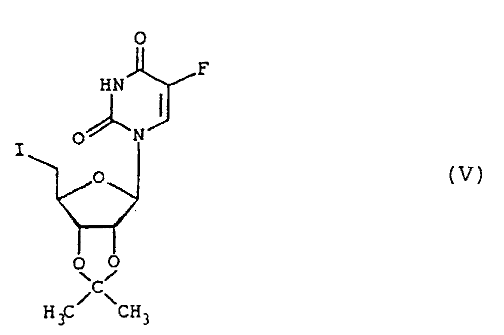 das folglich in sauren Medium erhalten wird; und
(d) Reduktion des 5'-Desoxy-5'-iod-5-fluoridin der Formel VI 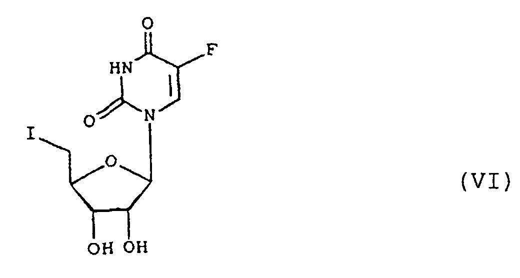 mit Wasserstoff oder einem Wasserstoffdonor.

2. Verfahren nach Anspruch 1, wobei in dem Derivat der Sulfonsäure der Formel III, R Methyl, Ethyl, n-Butyl, Trifluormethyl, Phenyl, einfach substituiertes Phenyl, d.h. mit einer Methyl-, Methoxy-, Nitrogruppe oder Halogen, zweifach substituiertes Phenyl, d.h. mit zwei Methylgruppen, oder dreifach substituiertes Phenyl, d.h. mit drei Methylgruppen, insbesondere 2,4,6-dreifachsubstituiertes Phenyl, ist.

3. Verfahren nach Anspruch 1, wobei in Schritt (a) ein Chlorid, ein Anhydrid oder ein gemischtes Anhydrid als ein funktionelles Derivat der Sulfonsäure der Formel III verwendet wird.

4. Verfahren nach Anspruch 3, wobei das funktionelle Derivat der Säure der Formel III ein Methansulfonylchlorid, ein Trifluormethansulfonylchlorid, ein Benzolsulfonylchlorid, ein p-Toluolsulfonylchlorid oder ein 2,4,6-Trimethylphenylsulfonylchlorid ist.

5. Verfahren nach Anspruch 1, wobei die Schritte (a), (b) und (c) ohne Isolierung der am Ende jedes der Schritte (a) und (b) erhaltenen Produkte durchgeführt werden.

6. Verfahren nach Anspruch 1, wobei die Reduktion des Schrittes (d) durch katalytische Hydrierung durchgeführt wird.

7. Verfahren nach Anspruch 6, wobei Palladium auf Aktivkohle als Katalysator verwendet wird.

8. Verfahren nach Anspruch 6, wobei die katalytische Hydrierung in der Gegenwart einer Base durchgeführt wird.

9. Verfahren nach Anspruch 8, wobei die Base ein sekundäres oder tertiäres Amin ist.

10. Verfahren nach Anspruch 9, wobei das sekundäre oder tertiäre Amin Trimethylamin, Triethylamin, Diisopropylamin, N-ethyldiisopropylamin, Pyridin, Dimethylaminopyridin, Morpholin, N-Methylmorpholin, 2-Picolin oder Chinolin ist.

11. Verfahren nach Anspruch 8, wobei die Base eine anorganische Base, bevorzugt Natrium- oder Kaliumhydrogencarbonat, ist.

12. Verfahren nach Anspruch 6, wobei Tributylzinnhydrid als ein Reduktionsmittel verwendet wird.

13. Verfahren nach Anspruch 12, wobei die Reduktion in der Gegenwart einer katalytischen Menge von α,α'-Azoisobutyronitril durchgeführt wird.

14. Verfahren nach Anspruch 6, wobei die Reduktion unter Verwendung von Cyclohexen oder Cyclohexadien als Wasserstoffdonor durchgeführt wird.

15. Verfahren nach Anspruch 14, wobei der Reduktionsschritt bei einer Temperatur zwischen 60°C und 90°C durchgeführt wird.

## Revendications

1. Procédé pour la préparation de la 5'-désoxy-5-fluorouridine de formule I: 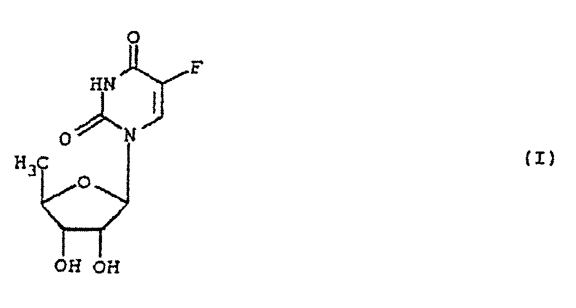 qui comprend :
(a) la réaction de la 2',3'-O-isopropylidène-5-fluorouridine de formule II: 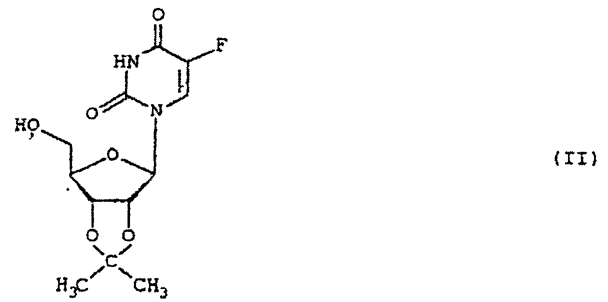 avec un dérivé fonctionnel d'un acide sulfonique de formule III
R-SO₃H (III)
dans laquelle R est un alkyle en C₁ à C₄, un trifluorométhyle, un groupe phényle non substitué, mono-, di- ou trisubstitué;
(b) la réaction du dérivé 5'-sulfonyloxy ainsi obtenu de formule IV : 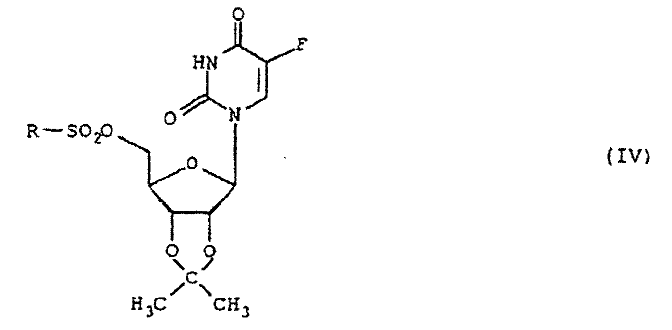 dans laquelle R est tel que défini ci-dessus, avec un iodure alcalin ou alcalino-terreux;
(c) l'hydrolyse de la 5'-désoxy-5'-iodo-2',3'-O-isopropylidène-5-fluorouridine de formule V 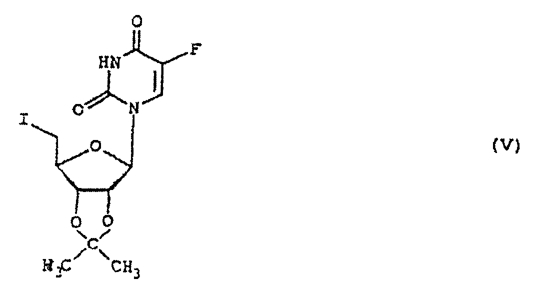 ainsi obtenue en milieu acide; et
(d) la soumission de la 5'-désoxy-5'-iodo-5-fluorouridine de formule VI : 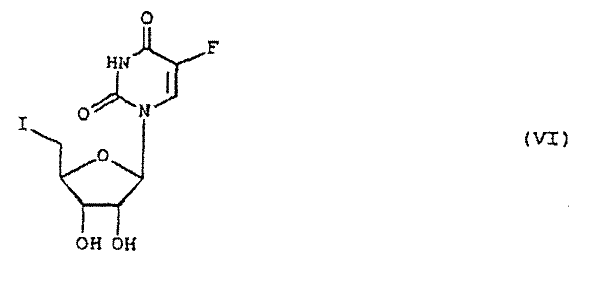 à une réduction avec l'hydrogène ou un donneur d'hydrogène.

2. Procédé selon la revendication 1, dans lequel, dans ledit dérivé d'acide sulfonique de formule III, R est un méthyle, un éthyle, un n-butyle, un trifluorométhyle, un phényle, un phényle monosubstitué, c'est-à-dire avec un méthyle, un méthoxy, un groupe nitro ou halogène, disubstitué, c'est-à-dire avec deux groupes méthyle, ou trisubstitué, c'est-à-dire avec trois groupes méthyle, en particulier 2,4,6-trisubstitués.

3. Procédé selon la revendication 1, dans lequel, dans l'étape (a), le chlorure, l'anhydrure ou un anhydrure mixte est utilisé comme dérivé fonctionnel de l'acide sulfonique de formule III.

4. Procédé selon la revendication 3, dans lequel le dérivé fonctionnel dudit acide de formule III est le chlorure de méthanesulfonyle, le chlorure de trifluorométhanesulfonyle, le chlorure de benzène-sulfonyle, le chlorure de p-toluènesulfonyle ou le chlorure de 2,4,6-triméhylphénylsulfonyle.

5. Procédé selon la revendication 1, dans lequel les étapes (a), (b) et (c) sont réalisées sans isoler les produits obtenus à la fin de chacune des étapes (a) et (b).

6. Procédé selon la revendication 1, dans lequel la réduction de l'étape (d) est réalisée par hydrogénation catalytique.

7. Procédé selon la revendication 6, dans lequel, du palladium sur charbon est utilisé comme catalyseur.

8. Procédé selon la revendication 6, dans lequel ladite hydrogénation catalytique est réalisée en présence d'une base.

9. Procédé selon la revendication 8, dans lequel ladite base est une amine secondaire ou tertiaire.

10. Procédé selon la revendication 9, dans lequel ladite amine secondaire ou tertiaire est la triméthylamine, la triéthylamine, la diisopropylamine, la N-éthyldiisopropylamine, la pyridine, la diméthyl-aminopyridine, la morpholine, la N-méthylmorpholine, la 2-picoline ou la quinoline.

11. Procédé selon la revendication 8, dans lequel ladite base est une base inorganique, de préférence le bicarbonate de sodium ou de potassium.

12. Procédé selon la revendication 6, dans lequel, comme agent réducteur, l'hydrure de tributyltyne est utilisé.

13. Procédé selon la revendication 12, dans lequel la réduction est réalisée en présence d'α,α'-azoisobutyronitrile, en quantité catalytique.

14. Procédé selon la revendication 6, dans lequel la réduction est réalisée en utilisant le cyclohexène ou le cyclohexadiène comme donneur d'hydrogène.

15. Procédé selon la revendication 14, dans lequel ladite étape de réduction est réalisée à une température comprise entre 60°C et 90°C.
